# EUROPÄISCHE PATENTSCHRIFT

(11) **EP 1 590 330 B1**
(45) Veröffentlichungstag und Bekanntmachung des Hinweises auf die Patenterteilung: **27.02.2013**
(21) Anmeldenummer: 04703168.7
(22) Anmeldetag: 19.01.2004
(51) Int. Cl.: C07D 231/16, A01N 43/56

(54) **PYRAZOLYLCARBOXANILIDE ZUR BEKÄMPFUNG VON UNERWÜNSCHTEN MIKROORGANISMEN**
PYRAZOLYL CARBOXANILIDES FOR CONTROLLING UNWANTED MICROORGANISMS
PYRAZOLYLCARBOXANILIDES POUR LUTTER CONTRE DES MICRO-ORGANISMES INDESIRABLES

(30) Priorität: 29.01.2003 DE 10303589
(43) Veröffentlichungstag der Anmeldung: 02.11.2005
(73) Patentinhaber: Bayer CropScience AG, 40789 Monheim (DE)
(72) Erfinder: DUNKEL, Ralf, 69001 Lyon (FR); ELBE, Hans-Ludwig, 42329 Wuppertal (DE); DAHMEN, Peter, 41470 Neuss (DE); WACHENDORFF-NEUMANN, Ulrike, 56566 Neuwied (DE); KUCK, Karl-Heinz, 40764 Langenfeld (DE); RIECK, Heiko., F-69110 Ste Foy les Lyon (FR)
(86) Internationale Anmeldenummer: PCT/EP2004/000344
(87) Internationale Veröffentlichungsnummer: WO 2004/067515

(56) Entgegenhaltungen:
- EP-A- 0 545 099
- EP-A- 0 589 301
- WO-A-02/08195
- WO-A-93/11117
- WO-A-03/010149

## Beschreibung

Die vorliegende Erfindung betrifft neue Pyrazolylcarboxanilide, mehrere Verfahren zu deren Herstellung und deren Verwendung zur Bekämpfung von unerwünschten Mikroorganismen.

Es ist bereits bekannt, dass zahlreiche Carboxanilide fungizide Eigenschaften besitzen (vergleiche WO 93-11117, EP-A 0 545 099, EP-A 0 589 301, WO 99/09013, DE 198 40 322, EP-A 0 824 099, JP 63048269). So lassen sich 1,3-Dimethyl-5-fluorpyrazol-4-carbonsäure-(2-cyclohexyl)-anilid, 1,3-Dimethyl-pyrazol-4-carbonsäure-(2-phenyl)-anilid und 1,3-Dimethyl-pyrazol-4-carbonsäure-[2-(2-fluor-phenyl)]-anilid zur Bekämpfung von Pilzen einsetzen. Die Wirksamkeit dieser Stoffe ist gut, lässt aber bei niedrigen Aufwandmengen in manchen Fällen zu wünschen übrig.

Es wurden nun neue Pyrazolylcarboxanilide der Formel (I) 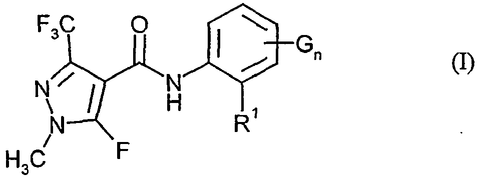 gefunden, in welcher
- R¹: für unsubstituiertes C₂-C₂₀-Akyl,

- G: für Halogen oder C₁-C₆-Alkyl steht,
- n: für 0, 1 oder 2 steht.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Weiterhin wurde gefunden, dass man Pyrazolylcarboxanilide erhält, indem man
a) Carbonsäure-Derivate der Formel (II) 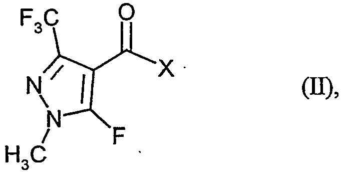 in welcher
   - X: für Halogen steht,
   mit einem Anilinderivat der Formel (III) 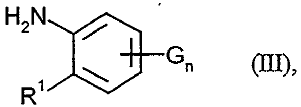 in welcher
   R¹, G und n die oben angegebenen Bedeutungen haben,
   gegebenenfalls in Gegenwart eines Säurebindemittels und gegebenenfalls in Gegenwart eines Verdünnungsmittels umsetzt, oder
b) Pyrazolylcarboxanilide der Formel (Ia) 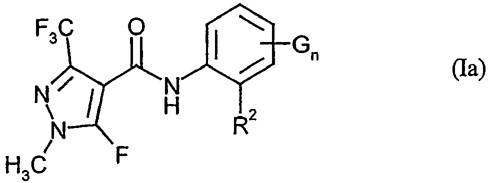 in welcher
   - G und n: die oben angegebenen Bedeutungen haben und
   - R²: für C₂-C₂₀-Alkenyl oder C₂-C₂₀-Alkinyl steht,
   gegebenenfalls in Gegenwart eines Verdünnungsmittels und gegebenenfalls in Gegenwart eines Katalysators hydriert, oder
c) Hydroxyalkylpyrazolylcarboxanilide der Formel (IV) 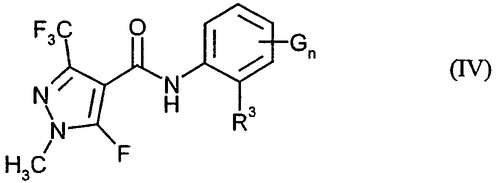 in welcher
   - G und n: die oben angegebenen Bedeutungen haben und
   - R³: für C₂-C₂₀-Hydroxyalkyl steht,
   gegebenenfalls in Gegenwart eines Verdünnungsmittels und gegebenenfalls in Gegenwart einer Säure dehydratisiert, oder
d) Halogenpyrazolylcarboxanilide der Formel (V) 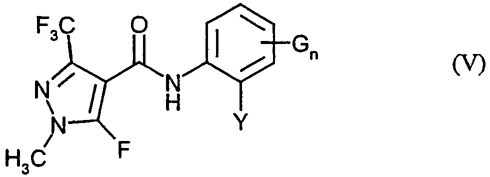 in welcher
   - G und n: die oben angegebenen Bedeutungen haben und
   - Y: für Brom oder Iod steht,
   mit einem Alkin der Formel (VI) 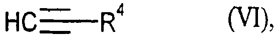 in welcher
   - R⁴: für C₂-C₁₈-Alkyl steht,
   oder einem Alken der Formel (VII) 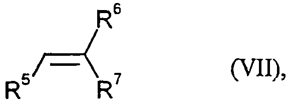 in welcher
   - R⁵, R⁶ und R⁷: unabhängig voneinander jeweils für Wasserstoff oder Alkyl stehen, und die Gesamtzahl der Kohlenstoffatome des offenkettigen Molekülteils die Zahl 20 nicht übersteigt,
   gegebenenfalls in Gegenwart eines Verdünnungsmittels, gegebenenfalls in Gegenwart eines Säurebindemittels und in Gegenwart eines oder mehrerer Katalysatoren umsetzt, oder
e) Ketone der Formel (VIII) 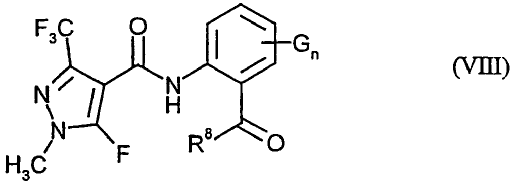 in welcher
   - G und n: die oben angegebenen Bedeutungen haben und
   - R⁸: für Wasserstoff oder C₁-C₁₈-Alkyl steht,
   mit einer Phosphorverbindung der allgemeinen Formel (X) 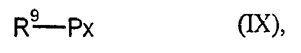 in welcher
   - R⁹: für Wasserstoff oder C₁-C₁₈-Alkyl steht,
   - Px: für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃ steht,
   gegebenenfalls in Gegenwart eines Verdünnungsmittels umsetzt.

Schließlich wurde gefunden, dass die neuen Pyrazolylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Pyrazolylcarboxanilide der Formel (I) eine wesentlich bessere fungizide Wirksamkeit als die konstitutionell ähnlichsten, vorbekannten Wirkstoffe gleicher Wirkungsrichtung.

Die erfindungsgemäßen Pyrazolylcarboxanilide sind durch die Formel (I) allgemein definiert.
- R¹: steht bevorzugt für unsubstituiertes C₂-C₁₂-Alkyl.
- R¹: steht besonders bevorzugt für verzweigtes, jeweils an beliebiger Stelle verknüpfte Ethyl, Propyl, Butyl, Pentyl, Hexyl, Heptyl, Octyl, Nonyl oder Decyl.
- G: steht besonders bevorzugt für Fluor, Chlor, Methyl, Ethyl, t-Butyl oder 2,4-Dimethylbutyl.

Weiterhin ganz besonders bevorzugt sind Verbindungen der Formel (I), in welcher
- R¹: für C₂-C₁₂-Alkyl, besonders bevorzugt C₂-C₆-Alkyl steht.

Weiterhin ganz besonders bevorzugt sind Verbindungen der Formel (I), in welcher
- n: für 0 steht.

Gesättigte Kohlenwasserstoffreste Alkyl können, soweit möglich, jeweils geradkettig oder verzweigt sein.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Die genannten Definitionen können untereinander in beliebiger Weise kombiniert werden. Außerdem können auch einzelne Definitionen entfallen.

Verwendet man 5-Fluor-1-methyl-3-(trifluormethyl)pyrazol-4-carbonyl-chlorid und 2-(1-Methylhexyl)anilin als Ausgangsstoffe, so kann das erfmdungsgemäße Verfahren a) durch das folgende Formelschema veranschaulicht werden:

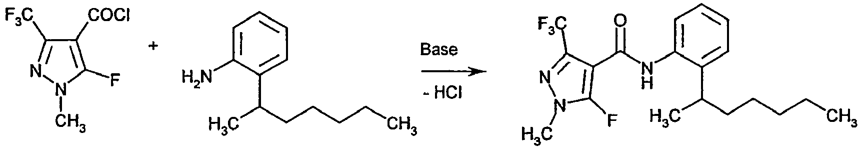

Die zur Durchführung des erfindungsgemäßen Verfahrens a) als Ausgangsstoffe benötigten Carbonsäure-Derivate sind durch die Formel (II) allgemein definiert. In dieser Formel (II) steht X bevorzugt für Chlor.

Die Carbonsäure-Derivate der Formel (II) sind bekannt und/oder lassen sich nach bekannten Verfahren herstellen (vgl. WO 93/11117, EP-A 0 545 099, EP-A 0 589 301 und EP-A 0 589 313).

Die zur Durchführung des erfindungsgemäßen Verfahrens a) als Ausgangsstoffe weiterhin benötigten Aniline sind durch die Formel (III) allgemein definiert. In dieser Formel (III) haben R¹, G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden.

Die Anilin-Derivate der Formel (III) sind bekannt und/oder lassen sich nach bekannten Methoden herstellen (vgl. z.B. Heterocycles (1989), 29(6), 1013-16; J. Med. Chem. (1996), 39(4), 892-903; Synthesis (1995), (6), 713-16; Synth. Commun. (1994), 24(2), 267-72; DE 2727416; Synthesis (1994), (2), 142-4; EP 0 824 099).

Verwendet man N-[2-((1Z)-1-Methylhex-1-enyl)phenyl][5-fluor-1-methyl-3-(trifluormethyl)pyrazol-4-yl]carboxamid und Wasserstoff als Ausgangsstoffe sowie einen Katalysator, so kann der Verlauf des erfindungsgemäßen Verfahrens b) durch das folgende Formelschema veranschaulicht werden:

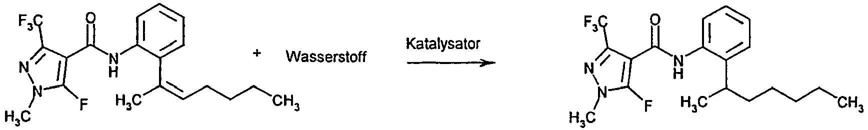

Die zur Durchführung des erfindungsgemäßen Verfahrens b) als Ausgangsstoffe benötigten Pyrazolylcarboxanilide sind durch die Formel (Ia) allgemein definiert. In dieser Formel (Ia) haben G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. als besonders bevorzugt für diese Reste angegeben wurden.
- R²: steht bevorzugt für C₂-C₁₂-Alkenyl oder C₂-C₁₂-Alkinyl.
- R²: steht besonders bevorzugt für jeweils geradkettiges oder verzweigtes, jeweils an beliebiger Stelle verknüpftes Ethenyl, Propenyl, Butenyl, Pentenyl, Hexenyl, Heptenyl, Octenyl, Nonenyl, Decenyl, Ethinyl, Propinyl, Butinyl, Pentinyl, Hexinyl, Heptinyl, Octinyl, Noninyl oder Decinyl.

Die Verbindungen der Formel (Ia) können nach Verfahren a), c), d) oder e) hergestellt werden.

Verwendet man [5-Fluor-1-methyl-3-(trifluormethyl)pyrazol-4-yl]-N-[2-(1-hydroxy-1-methylhexyl)phenyl]carboxamid als Ausgangsstoff sowie eine Säure, so kann der Verlauf des Verfahrens c) durch das folgende Formelschema veranschaulicht werden:

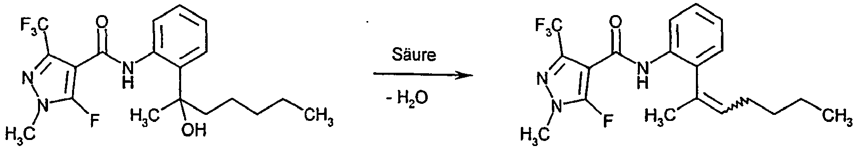

Die zur Durchführung des Verfahrens c) als Ausgangsstoffe benötigten Hydroxyalkylpyrazolylcarboxanilide sind durch die Formel (IV) allgemein definiert. In dieser Formel (IV) haben G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden.
- R³: steht bevorzugt für C₂-C₁₂- Hydroxyalkyl.
- R³: steht besonders bevorzugt für jeweils geradkettiges oder verzweigtes, jeweils an beliebiger Stelle verknüpftes Hydroxyethyl, Hydroxypropyl, Hydroxybutyl, Hydroxypentyl, Hydroxyhexyl, Hydroxyheptyl, Hydroxyoctyl, Hydroxynonyl oder Hydroxydecyl.

Die Verbindungen der Formel (IV) sind noch nicht bekannt und als neue Verbindungen ebenfalls Gegenstand der vorliegenden Anmeldung.

Es wurde auch gefunden, dass die Hydroxyalkylpyrazolylcarboxanilide der Formel (IV) sehr gute mikrobizide Eigenschaften besitzen und zur Bekämpfung unerwünschter Mikroorganismen sowohl im Pflanzenschutz als auch im Materialschutz eingesetzt werden können.

Die Hydroxyalkylpyrazolylcarboxanilide der Formel (TV) werden erhalten, indem man
f) Carbonsäure-Derivate der Formel (II) 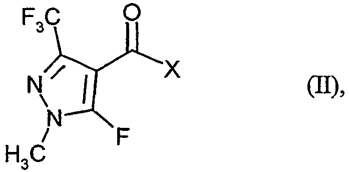 in welcher
   X die oben angegebenen Bedeutungen hat,
   mit einem Hydroayalkylanilinderivat der Formel (X) 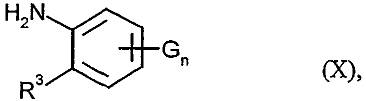 in welcher
   R³, G und n die oben angegebenen Bedeutungen haben,
   gegebenenfalls in Gegenwart eines Säurebindemittels und gegebenenfalls in Gegenwart eines Verdünnungsmittels umsetzt.

Verwendet man 5-Fluor-1-methyl-3(trifluormethyl)pyrazol-4-carbonyl-chlorid und 2-(2-Aminophenyl)-2-heptanol als Ausgangsstoffe, so kann der Verlauf des Verfahrens f) durch das folgende Formelschema veranschaulicht werden:

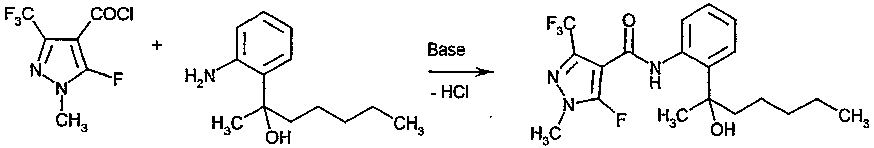

Die zur Durchführung des Verfahrens f) als Ausgangsstoffe benötigten Carbonsäure-Derivate der Formel (II) sind bereits weiter oben im Zusammenhang mit der Beschreibung des erfindungsgemäßen Verfahrens a) beschrieben worden.

Die zur Durchführung des Verfahrens f) als Ausgangsstoffe weiterhin benötigten Hydroxyalkylanilinderivate sind durch die Formel (X) allgemein definiert. In dieser Formel (X) haben R³, G und n bevorzugt bzw. besonders bevorzugt diejenigen Bedeutungen, die bereits im Zusammenhang mit der Beschreibung der erfindungsgemäßen Verbindungen der Formeln (I) bzw. (IV) als bevorzugt bzw. als besonders bevorzugt für diese Reste angegeben wurden.

Die Hydroxyalkylanilinderivate der Formel (X) sind bekannt und/oder können nach bekannten Methoden erhalten werden (vgl. z.B. US 3,917,592 oder EP 0 824 099).

Verwendet man [5-Fluor-1-methyl-3-(trifluormethyl)pyrazol-4-yl]-N-(2-iodphenyl)-carboxamid und 1-Pentin oder alternativ 1-Hexen als Ausgangsstoffe, sowie jeweils einen Katalysator und eine Base, so kann der Verlauf des Verfahrens d) durch die beiden folgenden Formelschemata veranschaulicht werden:

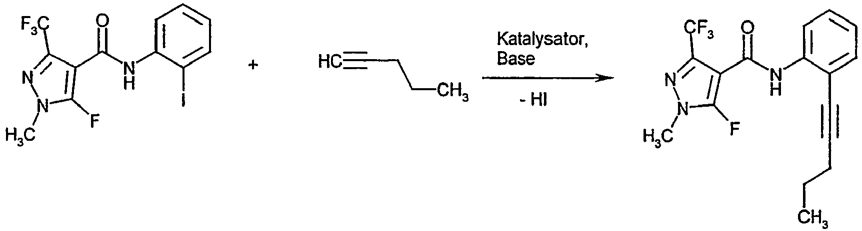

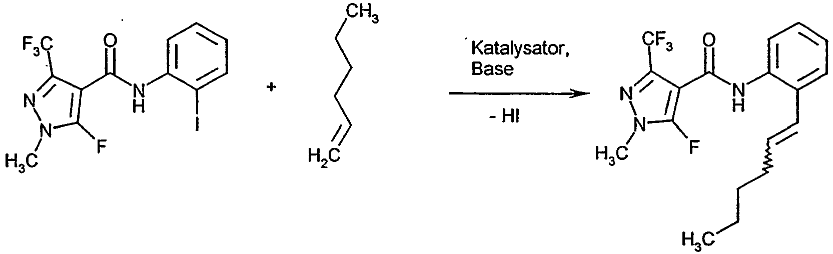

Die zur Durchführung des Verfahrens d) als Ausgangsstoffe benötigten Halogenpyrazolylcarboxanilide sind durch die Formel (V) allgemein definiert. In dieser Formel (V) haben G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden. Y steht bevorzugt für Brom oder Iod.

Sie werden erhalten, indem man
g) Carbonsäure-Derivate der Formel (II) 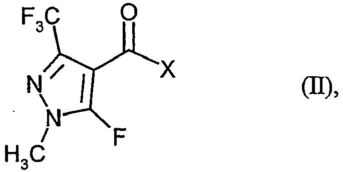 in welcher
   X die oben angegebenen Bedeutungen hat,
   mit einem Halogenanilin der Formel (XI) 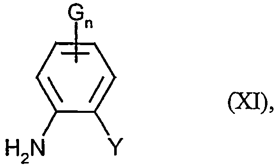 in welcher
   G, n und Y die oben angegebenen Bedeutungen haben,
   gegebenenfalls in Gegenwart eines Säurebindemittels und gegebenenfalls in Gegenwart eines Verdünnungsmittels umsetzt.

Verwendet man 5-Fluor-1-methyl-3-(trifluormethyl)pyrazol-4-carbonylchlorid und 2-Iodanilin als Ausgangsstoffe, so kann der Verlauf des erfindungsgemäßen Verfahrens g) durch das folgende Formelschema veranschaulicht werden:

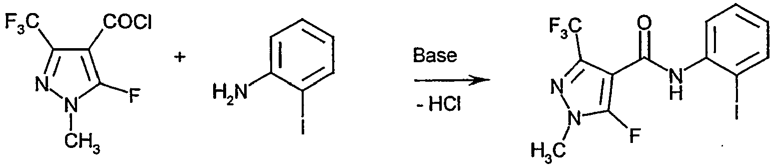

Die zur Durchführung des Verfahrens g) als Ausgangsstoffe benötigten Carbonsäure-Derivate der Formel (II) sind bereits weiter oben im Zusammenhang mit der Beschreibung des erfindungsgemäßen Verfahrens a) beschrieben worden.

Die zur Durchführung des Verfahrens g) als Ausgangsstoffe weiterhin benötigten Halogenaniline sind durch die Formel (XI) allgemein definiert. In dieser Formel (XI) haben G, n und Y bevorzugt bzw. besonders bevorzugt diejenigen Bedeutungen, die bereits im Zusammenhang mit der Beschreibung der erfindungsgemäßen Verbindungen der Formeln (I) bzw. (V) als bevorzugt bzw. besonders bevorzugt für diese Reste angegeben wurden.

Die Halogenaniline der Formel (XI) sind bekannte Synthesechemikalien.

Die zur Durchführung des Verfahrens d) weiterhin als Ausgangsstoffe benötigten Alkine sind durch die Formel (VI) allgemein definiert.
- R⁴: steht bevorzugt für C₂-C₁₀-Alkyl.
- R⁴: steht besonders bevorzugt für jeweils geradkettiges oder verzweigtes, jeweils an beliebiger Stelle verknüpftes Ethyl, Propyl, Butyl, Pentyl, Hexyl, Heptyl oder Octyl.

Die Alkine der Formel (VI) sind bekannte Synthesechemikalien.

Die zur Durchführung des Verfahrens d) weiterhin alternativ als Ausgangsstoffe benötigten Alkene sind durch die Formel (VII) allgemein definiert.
- R⁵, R⁶ und R⁷: stehen unabhängig voneinander bevorzugt jeweils für Wasserstoff oder Alkyl, wobei die Gesamtzahl der Kohlenstoffatome des offenkettigen Molekülteils die Zahl 12 nicht übersteigt.
- R⁵, R⁶ und R⁷: stehen unabhängig voneinander besonders bevorzugt jeweils für Wasserstoff jeweils geradkettiges oder verzweigtes, jeweils an beliebiger Stelle verknüpftes Ethyl, Propyl, Butyl, Pentyl, Hexyl, Heptyl oder Octyl, wobei die Gesamtzahl der Kohlenstoffatome des offenkettigen Molekülteils die Zahl 12 nicht übersteigt.

Die Alkene der Formel (VII) sind bekannte Synthesechemikalien.

Verwendet man N-(2-Acetylphenyl)[5-fluor-1-methyl-3-(trifluormethyl)pyrazol-4-yl]carboxamid und Butyl(triphenyl)-phosphonium-iodid als Ausgangsstoffe, so kann der Verlauf des Verfahrens e) durch das folgende Formelschema veranschaulicht werden:

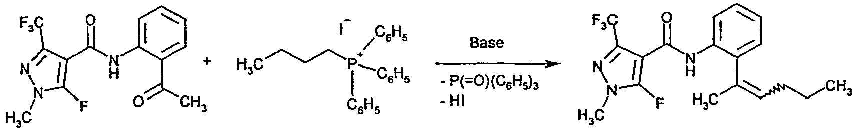

Die zur Durchführung des Verfahrens e) als Ausgangsstoffe benötigten Ketone sind durch die Formel (VIII) allgemein definiert. In dieser Formel (VIII) haben G und n bevorzugt bzw. besonders bevorzugt diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden.
- R⁸: steht bevorzugt für C₂-C₁₀-Alkyl.
- R⁸: steht besonders bevorzugt für jeweils geradkettiges oder verzweigtes, jeweils an beliebiger Stelle verknüpftes Ethyl, Propyl, Butyl, Pentyl, Hexyl, Heptyl oder Octyl.

Die Ketone der Formel (VIII) sind noch nicht bekannt. Sie sind als neue chemische Verbindungen ebenfalls Gegenstand der vorliegenden Anmeldung.

Sie werden erhalten, indem man
h) Carbonsäure-Derivate der Formel (II) 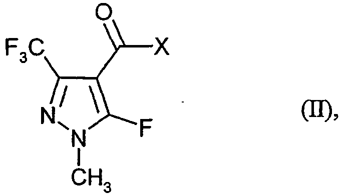 in welcher
   X die oben angegebenen Bedeutungen hat,
   mit Ketoanilinen der Formel (XII) 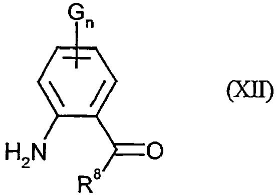 in welcher
   R⁸, G und n die oben angegebenen Bedeutungen haben,
   gegebenenfalls in Gegenwart eines Säurebindemittels und gegebenenfalls in Gegenwart eines Verdünnungsmittels umsetzt.

Verwendet man 5-Fluor-1-methyl-3-(trifluormethyl)pyrazol-4-carbonylchlorid und 1-(2-Aminophenyl)ethanon als Ausgangsstoffe, so kann der Verlauf des erfindungsgemäßen Verfahrens h) durch das folgende Formelschema veranschaulicht werden:

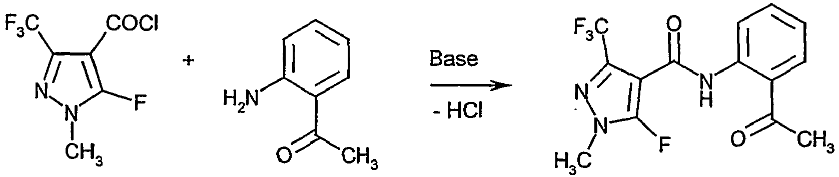

Die zur Durchführung des erfindungsgemäßen Verfahrens h) als Ausgangsstoffe benötigten Carbonsäure-Derivate der Formel (II) sind bereits weiter oben im Zusammenhang mit der Beschreibung des erfindungsgemäßen Verfahrens a) beschrieben worden.

Die zur Durchführung des Verfahrens h) weiterhin als Ausgangsstoffe benötigten Ketoaniline sind durch die Formel (XII) allgemein definiert. In dieser Formel (XII) haben R⁸, G und n bevorzugt bzw. besonders bevorzugt diejenigen Bedeutungen, die bereits im Zusammenhang mit der Beschreibung der erfindungsgemäßen Verbindungen der Formeln (I) bzw. (VIII) als bevorzugt bzw. besonders bevorzugt für diese Reste angegeben wurden.

Die Ketoaniline der Formel (XII) sind allgemein übliche Synthesechemikalien (vergleiche z. B. J. Am. Chem. Soc. 1978, 100, 4842-4857 oder US 4,032,573).

Die zur Durchführung des Verfahrens e) weiterhin als Ausgangsstoffe benötigten Phosphorverbindungen sind durch die Formel (IX) allgemein definiert.
- R⁹: steht bevorzugt für C₂-C₁₀-Alkyl.
- R¹¹: steht besonders bevorzugt für jeweils geradkettiges oder verzweigtes, jeweils an beliebiger Stelle verknüpftes Ethyl, Propyl, Butyl, Pentyl, Hexyl, Heptyl oder Octyl.
- Px: steht bevorzugt für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃.

Die Phosphorverbindungen der Formel (IX) sind bekannt und/oder können nach bekannten Verfahren hergestellt werden (vergleiche z. B. Justus Liebigs Ann. Chem. 1953, 580, 44-57 oder Pure Appl. Chem. 1964, 9, 307-335).

Als Verdünnungsmittel zur Durchführung der Verfahren a), f), g) und h) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die Verfahren a), f), g) und h) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der Verfahren a), f), g) und h) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens a) zur Herstellung der Verbindungen der Formel (I) setzt man pro mol des Carbonsäure-Derivates der Formel (II) im allgemeinen 0,2 bis 5 mol, vorzugsweise 0,5 bis 2 mol an Anilinderivat der Formel (III) ein.

Zur Durchführung des Verfahrens f) zur Herstellung der Verbindungen der Formel (IV) setzt man pro mol des Carbonsäure-Derivates der Formel (II) im allgemeinen 0,2 bis 5 mol, vorzugsweise 0,5 bis 2 mol an Hydroxyalkylanilinderivat der Formel (X) ein.

Zur Durchführung des Verfahrens g) zur Herstellung der Verbindungen der Formel (V) setzt man pro mol des Carbonsäure-Derivates der Formel (II) im allgemeinen 0,2 bis 5 mol, vorzugsweise 0,5 bis 2 mol an Halogenanilin der Formel (XI) ein.

Zur Durchführung des Verfahrens h) zur Herstellung der Verbindungen der Formel (VIII) setzt man pro mol des Carbonsäure-Derivates der Formel (II) im allgemeinen 0,2 bis 5 mol, vorzugsweise 0,5 bis 2 mol an Ketoanilin der Formel (XII) ein.

Als Verdünnungsmittel zur Durchführung des erfindungsgemäßen Verfahrens b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol oder Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether oder Diethylenglykolmonoethylether.

Das erfindungsgemäße Verfahren b) wird gegebenenfalls in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Die Hydrierung im erfindungsgemäßen Verfahren b) kann statt in Gegenwart von Wasserstoff in Kombination mit einem Katalysator auch in Anwesenheit von Triethylsilan durchgeführt werden.

Die Reaktionstemperaturen können bei der Durchführung des erfindungsgemäßen Verfahrens b) in einem größeren Bereich variiert werden. Im allgemeinen arbeitet man bei Temperaturen von 0°C bis 150°C, vorzugsweise bei Temperaturen von 0°C bis 80°C.

Als Verdünnungsmittel zur Durchführung des Verfahrens c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das Verfahren c) wird gegebenenfalls in Gegenwart einer Säure durchgeführt. Als solche kommen alle anorganischen und organischen Protonen- wie auch Lewissäuren, sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen-III-chlorid, Antimonpentachlorid, saure Ionenaustauscher, saure Tonerden und saures Kieselgel.

Die Reaktionstemperaturen können bei der Durchführung des Verfahrens c) in einem größeren Bereich variiert werden. Im allgemeinen arbeitet man bei Temperaturen von 0°C bis 150°C, vorzugsweise bei Temperaturen von 0°C bis 80°C.

Die Verfahren c) und b) können auch in einer Tandemreaktion ("Eintopf-Reaktion") durchgeführt werden. Dazu wird eine Verbindung der Formel (IV) gegebenenfalls in Gegenwart eines Verdünnungsmittels (geeignete Lösungsmittel wie für Verfahren c)), gegebenenfalls in Gegenwart einer Säure (geeignete Säuren wie für Verfahren c)) und in Anwesenheit von Triethylsilan umgesetzt.

Als Verdünnungsmittel zur Durchführung des Verfahrens d) kommen alle inerten organischen Lösungsmittel in Betracht. Hierzu gehören vorzugsweise Nitrile, wie Acetonitril, Propionitril, n- oder i-Butyronitril oder Benzonitril oder Amide, wie N,N-Dimethylformamid, N,N-Dimethylacetamid, N-Methylformanilid, N-Methylpyrrolidon oder Hexainethylphosphorsäuretriamid.

Das Verfahren d) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das Verfahren d) wird in Gegenwart eines oder mehrerer Katalysatoren durchgeführt.

Dazu eignen sich besonders Palladiumsalze oder -komplexe. Hierzu kommen vorzugsweise Palladiumchlorid, Palladiumacetat, Tetrakis-(triphenylphosphin)-Palladium oder Bis-(triphenylphosphin)-Palladiumdichlorid infrage. Es kann auch ein Palladiumkomplex in der Reaktionsmischung erzeugt werden, wenn man ein Palladiumsalz und ein Komplexligand getrennt zur Reaktion zugibt.

Als Liganden kommen vorzugsweise Organophosphorverbindungen infrage. Beispielhaft seien genannt: Triphenylphosphin, tri-o-Tolylphosphin, 2,2'-Bis(diphenylphosphino)-1,1'-binaphthyl, Dicyclohexylphosphinbiphenyl, 1,4-Bis(diphenylphosphino)butan, Bisdiphenylphosphinoferrocen, Di(tert.-butylphosphino)biphenyl, Di(cyclohexylphosphino)biphenyl, 2-Dieyclohexylphosphino-2'-NN-dimethylaminobiphenyl, Tricyclohexylphosphin, Tri-tert.-butylphosphin. Es kann aber auch auf Liganden verzichtet werden.

Das Verfahren d) wird ferner gegebenenfalls in Gegenwart eines weiteren Metallsalzes, wie Kupfersalzen, beispielsweise Kupfer-(I)-iodid durchgeführt.

Die Reaktionstemperaturen können bei der Durchführung des Verfahrens d) in einem größeren Bereich variiert werden. Im allgemeinen arbeitet man bei Temperaturen von 20°C bis 180°C, vorzugsweise bei Temperaturen von 50°C bis 150°C.

Zur Durchführung der Verfahrens d) zur Herstellung der Verbindungen der Formel (I) setzt man pro mol des Halogenpyrazolylcarboxanilids der Formel (V) im allgemeinen 1 bis 5 mol, vorzugsweise 1 bis 2 mol an Alkin der Formel (VI) oder Alken der Formel (VII) ein.

Als Verdünnungsmittel zur Durchführung des Verfahrens e) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoayethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether.

Das Verfahren e) wird gegebenenfalls in Gegenwart eines geeigneten Säureakzeptors durchgeführt. Als solche kommen alle üblichen starken Basen infrage. Hierzu gehören vorzugsweise Erdalkalimetall- oder Alkalimetallhydride, -hydroxide, -amide, -alkoholate oder Alkalimetall-Kohlenwasserstoffverbindungen, wie beispielsweise Natriumhydrid, Natriumhydroxid, Kaliumhydroxid, Natriumamid, Lithiumdiisopropylamid, Natrium-methylat, Natrium-ethylat, Kalium-tert.-butylat, Methyllithium, Phenyllithium oder Butyllithium.

Die Reaktionstemperaturen können bei der Durchführung des Verfahrens e) in einem größeren Bereich variiert werden. Im allgemeinen arbeitet man bei Temperaturen von -80°C bis 150°C, vorzugsweise bei Temperaturen von -30°C bis 80°C.

Zur Durchführung des Verfahrens e) zur Herstellung der Verbindungen der Formel (I) setzt man pro mol des Ketons der Formel (VIII) im allgemeinen 1 bis 5 mol, vorzugsweise 1 bis 2 mol an Phosphorverbindung der Formel (IX) ein.

Alle Verfahren werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Selerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Pyrenophora-Arten, von Krankheiten im Wein-, Obst- und Gemüseanbau, wie beispielsweise gegen Alternaria - oder Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften, in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.
Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dirnethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Irninoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)-pyridin; 2-Amino-4-methyl-N-phenyl-5-thiazol-carboxamid; 2-Chlor-N-(2,3-dihydro-1,1,3-trimethyl-1H-inden-4-yl)-3-pyridincarboxamid; 3,4,5-Trichlor-2,6-pyridindicarbonitril; Actinovate; cis-1-(4-Chlorphenyl)-2-(1H-1,2,4-triazol-1-yl)-cycloheptanol; Methyl-1-(2,3-dihydro-2,2-dimethyl-1H-inden-1-yl)-1H-imidazol-5-carboxylat; Monokaliumcarbonat; N-(6-Methoxy-3-pyridinyl)-cyclopro-pancarboxamid; N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin, Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-transisomer), Cyromazine, DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439, Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid, Imiprothrin, Indoxacarb, Iodofenphos, Iprobenfos, Isazofos, Isofenphos, Isoprocarb, Isoxathion, Ivermectin, Japonilure, Kadethrin, Kernpolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron, OK-5101, OK-5201, OK-9601, OK-9602, OK-9701, OK-9802, Omethoate, Oxamyl, Oxydemeton-methyl, Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb, Vamidothion, Vaniliprole, Verbutin, Verticillium lecanii, WL-108477, WL-40027, YI-5201, YI-5301, YI-5302, XMC, Xylylcarb, ZA-3274, Zeta-Cypermethrin, Zolaprofos, ZXI-8901, die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semicochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombinatin mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

### Herstellunizsbeispiele

### Beispiel 1

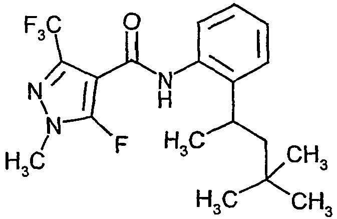

Eine Lösung bestehend aus 8,1 g (42,4 mmol) 5-Fluor-1-methyl-3-(trifluormethyl)-pyrazol-4-carbonyl-chlorid in 80 ml Tetrahydrofuran wird bei 0°C bis -10°C zu einer Lösung bestehend aus 6,5 g (28,3 mmol) des 2-(1,3,3-Trimethylbutyl)phenylamins und 5,7 g (56,6 mmol) Triethylamin in 200 ml Tetrahydrofuran getropft. Die Reaktionsmischung wird 1h bei 0°C gerührt. Zur Aufarbeitung wird über Kieselgel filtriert und aufkonzentriert. Reinigung an Kieselgel (Petrolether/Essigsäureethylester 3:1) liefert 10,1 g (94 % der Theorie) an [5-Fluor-1-methyl-3-(trifluormethyl)pyrazol-4-yl]-N-[2-(1,3,3-trimethylbutyl)phenyl]carboxamid mit dem log P (pH 2,3) = 4,14.

Analog Beispiel 1, sowie entsprechend den Angaben in den allgemeinen Verfahrensbeschreibungen, werden die in der nachstehenden Tabelle 1 genannten Verbindungen der Formel (I) erhalten.

| Bsp. Nr. | Verbindung | logP (pH 2,3) |
|---|---|---|
| 2 | 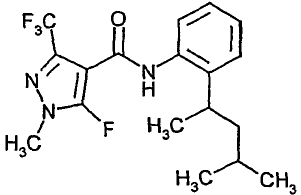 | 3,88 |
| 3 | 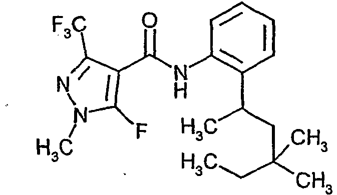 | 4,45 |
| 4 | 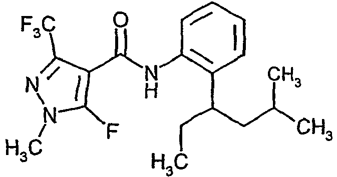 | 4,27 |
| 5 | 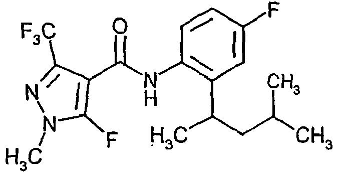 | 3,92 |

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele:

### Beispiel A

### Puccinia-Test (Weizen) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von *Puccinia recondita* besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Puccinia-Test (Weizen) / protektiv** | | |
|---|---|---|
| Wirkstoff Erfindungsgemäß | Aufwandmenge an Wirkstoff in g/ha | Wirkungsgrad in % |
| 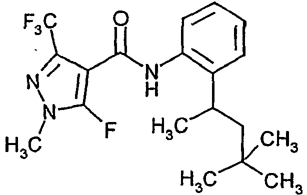 | 500 | 100 |
| 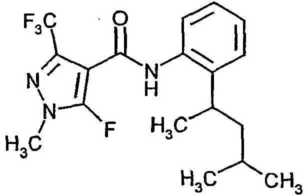 | 500 | 100 |

### Beispiel B

### Spaerotheca-Test (Gurke) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Sphaerotheca fuliginea* inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Spaerotheca-Test (Gurke) / protektiv** | | |
|---|---|---|
| Wirkstoff Erfindungsgemäß | Aufwandmenge an Wirkstoff in g/ha | Wirkungsgrad in % |
| 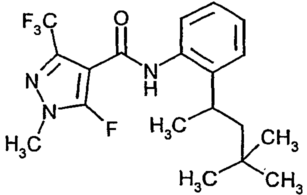 | 100 | 100 |
| 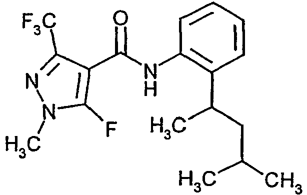 | 100 | 100 |
| 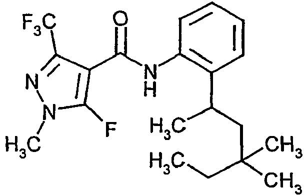 | 100 | 98 |

### Beispiel C

### Venturia - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt. 10 Tage nach der Inokulation erfolgt die Auswertung. Dabei bedeutet 0 % ein Wirkunggrad, der demjenigen der Kontrolle entspricht, während ein Wirkungsgrad von 100 % bedeutet, dass kein Befall beobachtet wird.

**Tabelle C**

| **Venturia - Test (Apfel) / protektiv** | | |
|---|---|---|
| Wirkstoff Erfindungsgemäß | Aufwandmenge an Wirkstoff in g/ha | Wirkungsgrad in % |
| 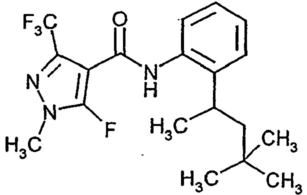 | 100 | 100 |
| 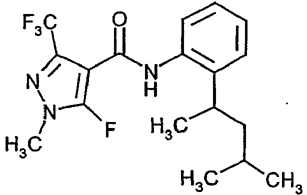 | 100 | 100 |
| 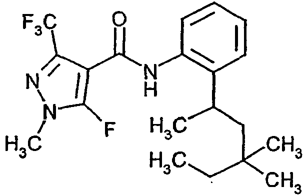 | 100 | 99 |

### Beispiel D

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| Wirkstoff Erfindungsgemäß | Aufwandmenge an Wirkstoff in g/ha | Wirkungsgrad in % |
|---|---|---|
| 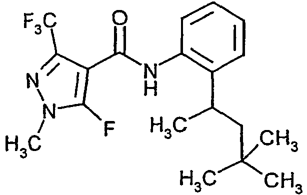 | 500 | 100 |
| 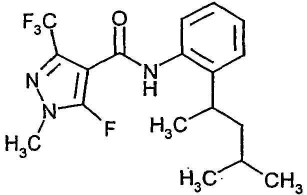 | 500 | 100 |
| 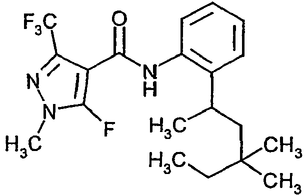 | 500 | 95 |

### Beispiel E

### In vitro-Test zur ED₅₀-Bestimmung bei Mikroorganismen

In die Kavitäten von Mikrotiterplatten wird eine methanolische Lösung des zu prüfenden Wirkstoffs, versetzt mit dem Emulgator PS16, pipettiert. Nachdem das Lösungsmittel abgedampft ist, werden je Kavität 200 µl Potatoe-Dextrose-Medium hinzugefügt.

Das Medium wurde vorher mit einer geeigneten Konzentration von Sporen bzw. Mycel des zu prüfenden Pilzes versetzt.

Die resultierenden Konzentrationen des Wirkstoffs betragen 0.1, 1, 10 und 100 ppm. Die resultierende Konzentration des Emulgators beträgt 300 ppm.

Die Platten werden anschließend 3-5 Tage auf einem Schüttler bei einer Temperatur von 22°C inkubiert, bis in der unbehandelten Kontrolle ein ausreichendes Wachstum feststellbar ist.

Die Auswertung erfolgt photometrisch bei einer Wellenlänge von 620 nm. Aus den Messdaten der verschiedenen Konzentrationen wird die Wirkstoffdosis, die zu einer 50%igen Hemmung des Pilzwachstums gegenüber der unbehandelten Kontrolle führt (ED₅₀), berechnet.

**Tabelle E**

| **In vitro-Test zur ED₅₀-Bestimmung bei Mikroorganismen** | | |
|---|---|---|
| Wirkstoff Erfindungsgemäß | Mikroorganimus | ED₅₀-Wert in ppm |
| 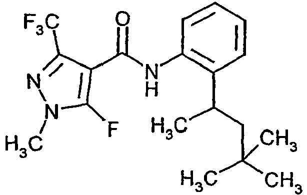 | Alternaria mali | < 0,1 |
| 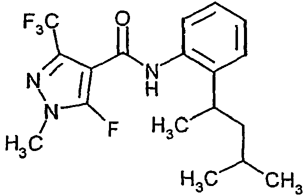 | Alternaria mali | 0,28 |
| 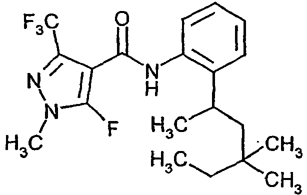 | Alternaria mali | < 0,1 |

## Patentansprüche

1. Pyrazolylcarboxanilide der Formel (I) 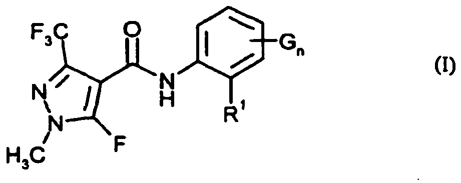 in welcher
R¹ für unsubstituiertes C₂-C₂₀-Alkyl,
G für Halogen oder C₁-C₆-Alkyl steht,
n für 0, 1 oder 2 steht

2. Pyrazolylcarboxanilide der Formel (I) gemäß Anspruch 1, in welcher
R¹ für unsubstituiertes C₂-C₁₂-Alkyl,
G für Halogen oder C₁-C₆-Alkyl steht,
n für 0, 1 oder 2 steht

3. Pyrazolylcarboxanilide der Formel (I) gemäß Anspruch 1, in welcher
R¹ für unsubstituiertes C₂-C₆-Alkyl,
G für Fluor, Chlor, Methyl, Ethyl, t-Butyl oder 2,4-Dimethylbutyl steht,
n für 0, 1 oder 2 steht.

4. Pyrazolylcarboxanilide der Formel (I) gemäß einem oder mehreren der Ansprüche 1 bis 3, in welcher n für 0 steht.

5. Verfahren zum Herstellen der Verbindungen der Formel (I) gemäß Anspruch 1, **dadurch gekennzeichnet, dass** man
a) Carbonsäure-Derivate der Formel (II) 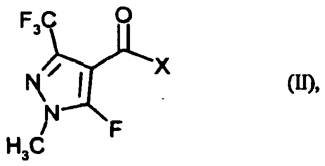 in welcher
X für Halogen steht,
mit einem Anilinderivat der Formel (III) 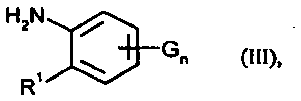 in welcher
R¹, G und n die oben angegebenen Bedeutungen haben,
gegebenenfalls in Gegenwart eines Säurebindemittels und gegebenenfalls in Gegenwart eines Verdünnungsmittels umsetzt, oder
b) Pyrazolylcarboxanilide der Formel (Ia) 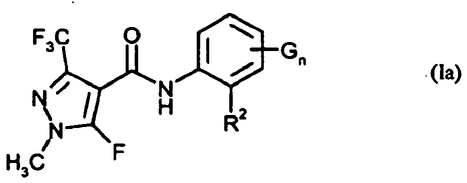 in welcher
G und n die oben angegebenen Bedeutungen haben und
R² für C₂-C₂₀-Alkenyl oder C₂-C₂₀-Alkinyl steht,
gegebenenfalls in Gegenwart eines Verdünnungsmittels und gegebenenfalls in Gegenwart eines Katalysators hydriert.

6. Mittel zum Bekämpfen unerwünschter Mikroorganismen, **gekennzeichnet durch** einen Gehalt an mindestens einem Pyrazolylcarboxanilid der Formel (I) gemäß Anspruch 1 neben Streckmitteln und/oder oberflächenaktiven Stoffen.

7. Verwendung von Pyrazolylcarboxaniliden der Formel (I) gemäß Anspruch 1 zum Bekämpfen unerwünschter Mikroorganismen im Pflanzenschutz und im Materialschutz

8. Verfahren zum Bekämpfen unerwünschter Mikroorganismen im Pfasenschtz im Materialschutz, **dadurch gekennzeichnet, dass** man Pyrazolylcarboxanilide der Formel (I) gemäß Anspruch 1 auf die Mikroorganismen und/oder deren Lebensraum ausbringt.

9. Verfahren zum Herstellen von Mitteln zum Bekämpfen unerwünschter Mikroorganismen, **dadurch gekennzeichnet, dass** man Pyrazolylcarboxanilide der Formel (I) gemäß Anspruch 1 mit Streckmitteln und/oder oberflächenaktiven Stoffen vermischt.

10. Hydroxyalkylpyrazolylcarboxanilide der Formel (IV) 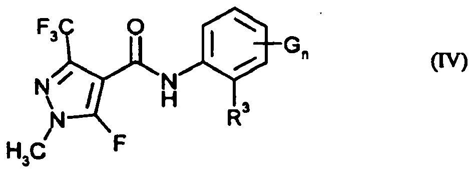 in welcher
G und n die in einem oder mehreren der Ansprüche 1 bis 4 angegebenen Bedeutungen haben und
R³ für C₁-C₂₀-Hydroxyalkyl steht.

11. Ketone der Formel (VIII) 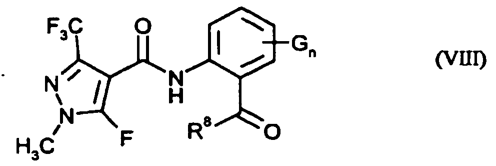 in welcher
G und n die in einem oder mehreren der Ansprüche 1 bis 4 angegebenen Bedeutungen haben und
R⁸ für Wasserstoff C₁-C₁₈-Alkyl steht.

## Claims

1. Pyrazolylcarboxanilides of the formula (I) 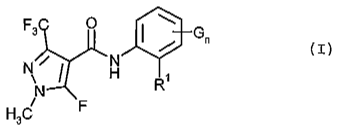 in which
R¹ represents unsubstituted C₂-C₂₀-alkyl
G represents halogen or C₁-C₆-alkyl,
n represents 0, 1 or 2.

2. Pyrazolylcarboxanilides of the formula (I) according to Claim 1, in which
R¹ represents unsubstituted C₂-C₁₂-alkyl
G represents halogen or C₁-C₆-alkyl,
n represents 0, 1 or 2.

3. Pyrazolylcarboxanilides of the formula (I) according to Claim 1, in which
R¹ represents unsubstituted C₂-C₆-alkyl,
G represents fluorine, chlorine, methyl, ethyl, t-butyl or 2,4-dimethylbutyl,
n represents 0, 1 or 2.

4. Pyrazolylcarboxanilides of the formula (I) according to one or more of Claims 1 to 3, in which n represents 0.

5. Process for preparing the compounds of the formula (I) according to Claim 1, **characterized in that**
a) carboxylic acid derivatives of the formula (II) 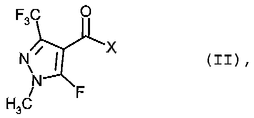 in which
X represents halogen,
are reacted with an aniline derivative of the formula (III) 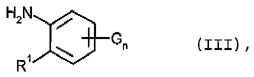 in which
R¹, G and n are as defined above,
if appropriate in the presence of an acid binder and if appropriate in the presence of a diluent, or
b) pyrazolylcarboxanilides of the formula (Ia) 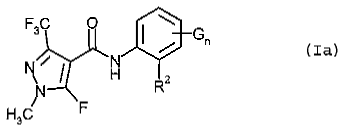 in which
G and n are as defined above and
R² represents C₂-C₂₀-alkenyl or C₂-C₂₀-alkynyl are hydrogenated, if appropriate in the presence of a diluent and if appropriate in the presence of a catalyst.

6. Composition for controlling unwanted microorganisms, **characterized in that** it comprises at least one pyrazolylcarboxanilide of the formula (I) according to Claim 1, in addition to extenders and/or surfactants.

7. Use of pyrazolylcarboxanilides of the formula (I) according to Claim 1 for controlling unwanted microorganisms in crop protection and in the protection of materials.

8. Method for controlling unwanted microorganisms in crop protection and in the protection of materials, **characterized in that** pyrazolylcarboxanilides of the formula (I) according to Claim 1 are applied to the microorganisms and/or their habitat.

9. Process for preparing compositions for controlling unwanted microorganisms, **characterized in that** pyrazolylcarboxanilides of the formula (I) according to Claim 1 are mixed with extenders and/or surfactants.

10. Hydroxyalkylpyrazolylcarboxanilides of the formula (IV) 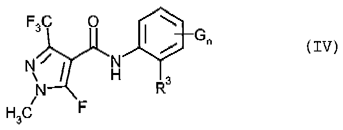 in which
G and n are as defined in one or more of Claims 1 to 4 and
R³ represents C₂-C₂₀-hydroxyalkyl.
11. Ketones of the formula (VIII) 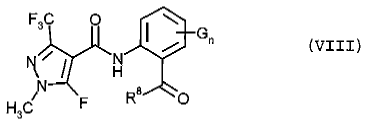 in which
G and n are as defined in one or more of Claims 1 to 4 and
R⁸ represents hydrogen or C₁-C₁₈-alkyl.

## Revendications

1. Pyrazolylcarboxanilides de formule (1) 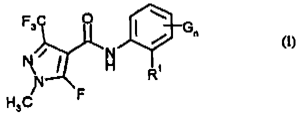 dans laquelle
R¹ représente C₂-C₂₀-alkyle non substitué,
G représente halogène ou C₁-C₆-alkyle,
n vaut 0, 1 ou 2.

2. Pyrazolylcarboxanilides de formule (I) selon la revendication 1, dans laquelle
R¹ représente C₂-C₁₂-alkyle non substitué,
G représente halogène ou C₁-C₆-alkyle,
n vaut 0, 1 ou 2.

3. Pyrazolylcarboxanilides de formule (I) selon la revendication 1, dans laquelle
R¹ représente C₂-C₆-alkyle non substitué,
G représente fluor, chlore, méthyle, éthyle, t-butyle ou 2,4-diméthylbutyle,
n vaut 0, 1 ou 2.

4. Pyrazolylcarboxanilides de formule (I) selon l'une ou plusieurs des revendications 1 à 3, dans laquelle n vaut 0.

5. Procédé pour la préparation des composés de formule (I) selon la revendication 1, **caractérisé en ce qu'**on
a) transforme des dérivés d'acide carboxylique de formule (II) 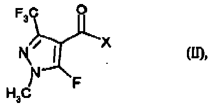 dans laquelle
X représente halogène,
avec un dérivé d'aniline de formule (III) 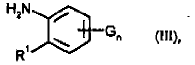 dans laquelle
R¹, G et n présentent les significations indiquées ci-dessus,
le cas échéant en présence d'un liant d'acide et le cas échéant en présence d'un diluant, ou
b) on hydrogène des pyrazolylcarboxanilides de formule (Ia) 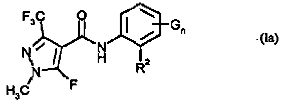 dans laquelle
G et n présentent les significations indiquées ci-dessus, et
R² représente C₂-C₂₀-alcényle ou C₂-C₂₀-alcynyle, le cas échéant en présence d'un diluant et le cas échéant en présence d'un catalyseur.

6. Agent pour lutter contre des microorganismes non souhaités, **caractérisé par** une teneur en au moins un pyrazolylcarboxanilide de formule (I) selon la revendication 1, outre des agents d'allongement et/ou des substances tensioactives.

7. Utilisation de pyrazolylcarboxanilides de formule (I) selon la revendication 1 pour lutter contre des microorganismes non souhaités dans la protection des végétaux et des matériaux.

8. Procédé pour lutter contre des microorganismes non souhaités dans la protection des plantes et des matériaux, **caractérisé en ce qu'**on épand des pyrazolylcarboxanilides de formule (I) selon la revendication 1 sur les microorganismes et/ou leur espace de vie.

9. Procédé pour préparer des agents pour lutter contre des microorganismes non souhaités, **caractérisé en ce qu'**on mélange des pyrazolylcarboxanilides de formule (I) selon la revendication 1 avec des agents d'allongement et/ou des substances tensioactives.

10. Hydroxyalkylpyrazolylcarboxanilides de formule (IV) 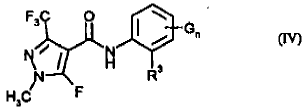 dans laquelle
G et n présentent les significations indiquées dans une ou plusieurs des revendications 1 à 4 et
R³ représente C₂-C₂₀-hydroxyalkyle.
11. Cétones de formule (VIII) 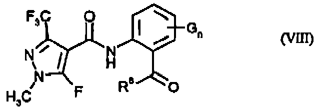 dans laquelle
G et n présentent les significations indiquées dans une ou plusieurs des revendications 1 à 4 et
R^{e} représente hydrogène ou C₁-C₁₈-alkyle.
